# FASCICULE DE BREVET EUROPEEN

(11) **EP 1 681 991 B1**
(45) Date de publication et mention de la délivrance du brevet: **22.12.2010**
(21) Numéro de dépôt: 04817095.5
(22) Date de dépôt: 04.10.2004
(51) Int. Cl.: A61B 3/10, G02C 13/00

(54) **DISPOSITIF DE DETERMINATION DE LA DISTANCE ENTRE LE GLOBE OCULAIRE ET LA FACE INTERNE D`UN VERRE CORRECTEUR**
VORRICHTUNG ZUR BESTIMMUNG DES ABSTANDS ZWISCHEN DEM AUGAPFEL UND DER INNENFLÄCHE EINER KORRIGIERENDEN LINSE
DEVICE FOR DETERMINING THE DISTANCE BETWEEN THE EYE BALL AND THE INTERNAL FACE OF A CORRECTIVE LENS

(30) Priorité: 03.10.2003 FR 0311613
(43) Date de publication de la demande: 26.07.2006
(73) Titulaire: ACEP FRANCE, 75008 Paris (FR)
(72) Inventeur: SAYAG, Jean-Philippe, F-75008 Paris (FR)
(74) Mandataire: Puiroux, Guy
(86) Numéro de dépôt international: PCT/FR2004/002500
(87) Numéro de publication internationale: WO 2005/032359

(56) Documents cités:
- DE-U- 1 925 995
- FR-A- 2 474 177
- FR-A- 2 558 714
- FR-A- 2 690 833

## Description

La présente invention concerne un dispositif destiné à déterminer la distance existant entre le globe oculaire d'un oeil d'un patient et la face interne des verres correcteurs de celui-ci et à contrôler l'inclinaison de ces derniers.

On connaît, dans le domaine de la lunetterie, différents procédés et appareils destinés à déterminer les paramètres physiologiques d'un patient en regard d'une monture de lunettes. Certains de ces appareils permettent d'assurer l'enregistrement automatique des mesures au moyen d'une caméra et la détermination des mesures appropriées résultantes à l'aide de moyens de traitement des images obtenues. Les mesures effectuées permettent ensuite à un opticien d'assurer un positionnement optimal des verres correcteurs sur la monture en fonction des yeux du patient.

Afin d'être en mesure d'exploiter, au maximum de leurs possibilités, certains nouveaux types de verres mis récemment sur le marche, l'opticien est contraint de mesurer d'une part la distance existant entre la partie frontale de chacun des globes oculaires du patient et la face interne de chaque verre de lunettes et d'autre part le bon positionnement angulaire des verres par rapport à la verticale.

Cette mesure a imposé jusqu'à présent de réaliser une première prise de vue de face, puis ensuite une seconde prise de vue de profil du patient.

Le document DE 1 925 995 U révèle un dispositif de saisie de l'écartement existant entre la partie frontale du globe oculaire de l'oeil d'un patient et la face interne d'un verre correcteur fixé sur une monture de lunettes portée par le dit patient, comprenant:
- un dispositif optique destinée à saisir une vue frontale de la monture chaussée par le patient;
- un élément positionneur, destiné à être fixé à ladite monture,
- le dit élément positionneur étant muni sur un côté de moyens de renvoi, destinés à renvoyer l'image transversale du dit écartement en direction du dispositif optique.

La présente invention a pour but de proposer un dispositif permettant de réaliser les mesures habituelles de face et de profil par une seule et même prise de vue unique.

La présente invention a ainsi pour objet un dispositif défini dans la revendication 1.

Dans un mode de mise en oeuvre de la présente invention, les moyens de renvoi pourront être fixés sur l'un des côtés latéraux de l'élément positionneur, et préférentiellement à droite ou à gauche de celui-ci. Ils pourront être constitués d'un miroir plan incliné d'un angle voisin de 45° par rapport au plan de la monture de lunettes, de façon à renvoyer une image transversale dudit écartement vers l'objectif de la caméra. Le miroir pourra, notamment pour des raisons de coût ou d'encombrement, être constitué d'un matériau de synthèse. Il pourra également être fixé de façon amovible sur l'élément positionneur.

Ces moyens de renvoi pourront également être constitués d'un prisme à réflexion totale, d'un angle au sommet voisin de 45° par rapport au plan de la monture de lunettes.

Suivant l'invention l'élément positionneur sera préférentiellement pourvu de deux repères décalés dans un sens perpendiculaire à la face frontale de l'élément positionneur, à savoir un repère antérieur et un repère postérieur. Le repère postérieur sera notamment disposé sur la face frontale de l'élément positionneur.

Les repères antérieur et postérieur seront préférentiellement tels qu'ils permettront de caractériser et mesurer au moins leur décalage dans le sens vertical et éventuellement leur décalage dans le sens horizontal, et pourront ainsi être notamment constitués par des croix. Un tel décalage, une fois mesuré dans le sens vertical permettant d'obtenir par un calcul trigonométrique simple l'angle d'inclinaison de la monture par rapport à la verticale. De même le décalage dans le sens horizontal permettant d'obtenir l'angle d'inclinaison de la monture par rapport à l'horizontale

La présente invention se révèle également particulièrement intéressante en raison de sa simplicité de mise en oeuvre qui se traduit à la fois sur le plan de l'efficacité des résultats et sur celui des coûts de la mise en oeuvre.

Par ailleurs la simplicité et la légèreté du dispositif suivant l'invention permettent à l'utilisateur de conserver un port de tête naturel qui n'est pas perturbé par un appareil de fort volume contre lequel l'utilisateur doit mettre son visage en appui. La mesure effectuée est donc une mesure "naturelle" correspondant au port de tête habituel du patient.

On décrira ci-après, à titre d'exemple non limitatif, une forme d'exécution de la présente invention, en référence au dessin annexé sur lequel :
- La figure 1 est une vue de face représentant un patient dont les lunettes sont équipées d'un dispositif suivant l'invention.
- La figure 2 est une vue de dessus schématique représentant les éléments de la figure 1.
- La figure 2a est une vue de détails partielle et agrandie de la figure 2.
- La figure 3 est une vue de face d'une première variante de mise en oeuvre du dispositif suivant l'invention.
- La figure 4 est une vue partielle d'un second mode de mise en oeuvre de l'invention.

La figure 5 est une vue partielle frontale d'un mode de mise en oeuvre préférentiel de la présente invention.

La figure 6 est une vue partielle frontale agrandie de la figure 5.

La figure 7 est une vue partielle en perspective et agrandie de la figure 5.

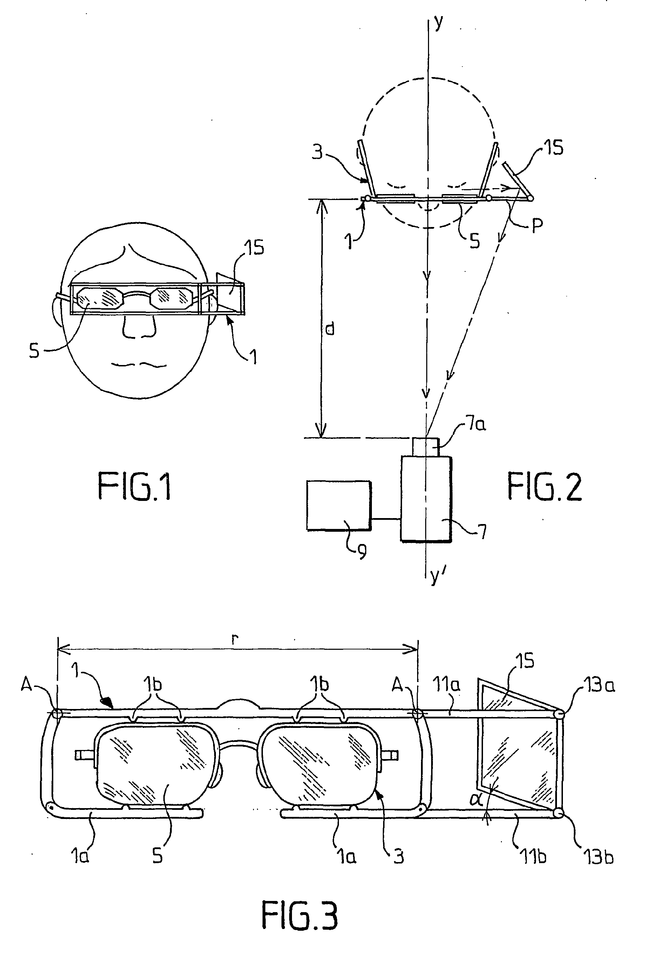

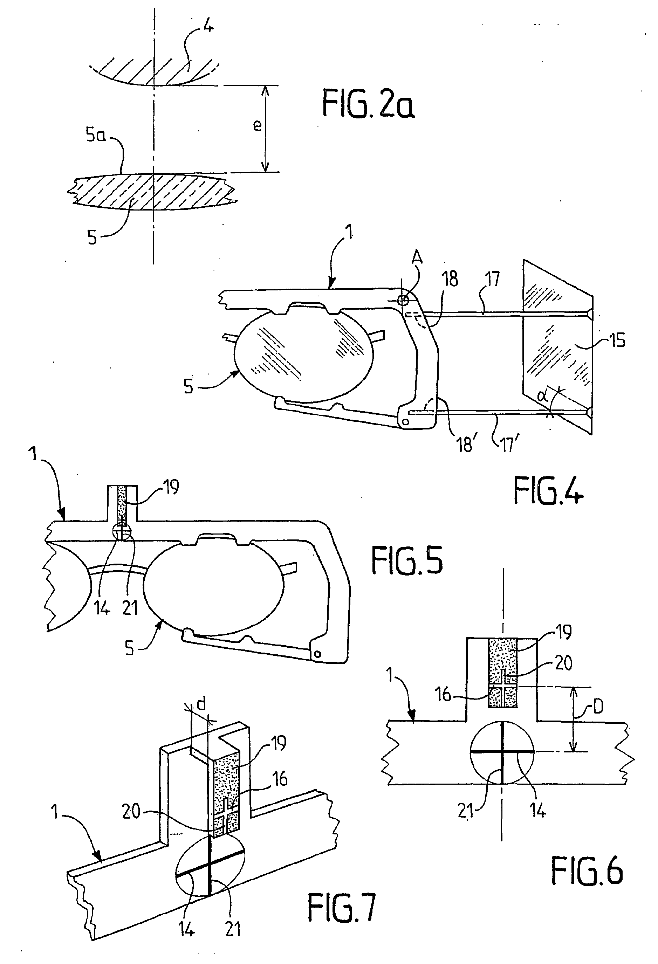

On a représenté sur les figures 1 et 2 un dispositif de saisie des paramètres physiologiques d'un patient nécessaires au positionnement et au réglage correct de verres 5 à l'intérieur d'une monture de lunettes 3.

Ce dispositif est constitué d'un élément positionneur 1 qui est fixé sur la monture de lunettes 3, celle-ci étant chaussée par le patient ainsi que représenté sur les figures 1 et 2, et d'une caméra de prise d'images 7 dont l'axe yy' est disposé sensiblement perpendiculairement au plan P de l'élément positionneur 1 et qui est associée à des moyens de saisie d'images 9, constitués notamment, par exemple, d'un micro-ordinateur.

La fixation de la monture de lunettes 3 sur l'élément positionneur 1 s'effectue par des branches latérales 1a dont est muni l'élément positionneur 1 qui sollicitent élastiquement la base de la monture 3 et l'appliquent contre des crochets supérieurs de maintien 1b, ce qui permet d'assurer un positionnement précis de la monture 3 par rapport à l'élément positionneur 1.

L'objectif 7a de la caméra 7 est disposé à une distance d de l'élément positionneur 1 qui est de l'ordre d'environ 300 cm. Dans ces conditions, on comprend qu'à partir de l'image de la monture fournie par la caméra de prise de vue 7, il est possible, la longueur d étant connue, d'en déduire la valeur de toute distance au niveau du plan P de la monture 3. Plus simplement on peut réaliser sur l'élément positionneur 1 des repères A écartés d'une distance r connue permettant, sans qu'il soit nécessaire de connaître la distance d séparant l'objectif 7a de la caméra de l'élément positionneur 1, de déterminer par une simple mesure de cette distance sur l'image obtenue et un simple calcul, toute distance existant dans le plan P.

Comme mentionné précédemment, dans le cadre de la mise en oeuvre de certains types de verres correcteurs, il est nécessaire, d'une part, de réaliser la mesure de l'écartement e qui sépare la partie externe du globe oculaire 4 de l'oeil d'un patient de la face interne 5a d'un verre 5 correspondant et, d'autre part, de contrôler le bon positionnement angulaire du bord des verres par rapport au plan vertical. Dans l'état antérieur de la technique, on réalise cette mesure, soit en faisant appel à une seconde caméra dont l'axe de prise de vue transversal est perpendiculaire à l'axe yy' de prise de vue de la première caméra 7, soit en utilisant cette dernière après avoir fait pivoter la tête du patient de 90°, de façon à amener son axe de prise de vue suivant l'axe transversal.

Pour obtenir suivant la présente invention sur une seule et même image la vue frontale classique de la monture 3 équipée du positionneur 1, et la vue transversale montrant l'écartement e séparant la partie antérieure du globe oculaire de la face interne 5a des verres 5 disposés sur la monture 3, l'élément positionneur 1 est prolongé, sur l'un de ses côtés, de préférence à droite ou à gauche de l'élément positionneur (à droite sur la figure 3) par deux bras 11a et 11b sensiblement parallèles et dont les extrémités respectives 13a et 13b assurent le maintien d'un miroir plan 15 dont la surface est inclinée d'un angle α par rapport au plan P de l'élément positionneur 1. Cet angle α, qui est voisin de 45°, est tel qu'il renvoie vers l'objectif 7a de la caméra 7 l'image transversale souhaitée et qui peut ainsi être captée par celle-ci en même temps ou sensiblement, que l'image frontale, et traitée ensuite par tout moyen de traitement d'images approprié sans qu'il soit nécessaire pour cela de faire appel à une seconde caméra ou de solliciter la rotation de la tête du patient.

On peut bien entendu suivant l'invention prolonger l'élément positionneur vers le haut ou vers le bas, mais on a constaté que, dans le cas de certains patients, la morphologie de ces derniers rendait la mesure de la distance e plus difficile.

Les moyens d'analyse et de traitement d'images pourront ainsi calculer l'écartement e souhaité en comparant celui-ci à la distance x connue séparant les repères A de l'élément positionneur 1 ainsi que le praticien opère habituellement dans l'état antérieur de la technique, en appliquant éventuellement une correction due à la distance supplémentaire de parcours des rayons lumineux.

On a constaté que la présente invention pouvait également être utilisée afin de contrôler le bon positionnement de la monture dans le plan vertical. En effet tout décalage de la ligne formée par le bord de la monture dans un sens ou dans un autre se détecte facilement et peut même faire l'objet de mesure.

On pourra, dans un mode de mise en oeuvre particulièrement intéressant de l'invention qui est représenté sur les figures 5 à 7, contrôler également le positionnement de la monture 5 dans le sens vertical et mesurer l'inclinaison de celle-ci en disposant un repère horizontal 14 sur l'élément positionneur 1 et un second repère horizontal 16 sur un doigt 19 qui est écarté de l'élément positionneur et disposer en avant de celui-ci d'une distance déterminée d. On comprend que l'écartement apparent D existant entre les deux repères horizontaux 14 et 16, qui est vu par la caméra 7, dépend de l'inclinaison de la monture 5 par rapport à la verticale et que la simple mesure de cette distance D permet de calculer par une simple opération trigonométrique ladite inclinaison.

Préférentiellement suivant l'invention, on fera en sorte que, à l'équilibre, c'est-à-dire pour une inclinaison nulle de la monture 5 par rapport à la verticale les deux repères 14 et 16 soient décalés d'une certaine distance D donnée, ce qui facilite leur repérage, en évitant que le repère 14 se trouve masqué par le support du repère 16, à savoir le doigt 19.

On pourra également, ainsi que représenté sur les figures 5 à 7 réaliser également des repères verticaux respectifs 20 et 21 sur le doigt 19 et sur l'élément positionneur 1. Les repères permettront de la même façon de contrôler et calculer l'angle formé par le plan de l'élément positionneur 1 avec le plan vertical contenant l'axe yy'.

Dans un autre mode de mise en oeuvre de la présente invention, qui est représentée sur la figure 4, les moyens de renvoi sont constitués d'un miroir réalisé dans une plaquette de matière de synthèse, (ce qui présente les avantages de rendre ce miroir particulièrement léger et bon marché) qui est pourvue à sa partie supérieure et à sa partie inférieure de branches respectives 17 et 17', qui sont liées au miroir de façon que celui-ci forme l'angle α souhaité, et qui viennent prendre place dans des trous parallèles 18 et 18' prévus dans les parties supérieure et inférieure de l'élément positionneur 1.

Un tel mode de mise en oeuvre est intéressant en ce qu'il permet de rendre l'élément positionneur démontable, le praticien ne mettant celui-ci en place sur l'élément positionneur 1 que lorsqu'il souhaite réaliser la mesure de la distance e existant entre le globe oculaire d'un oeil du patient et la face interne 5a des verres, et/ou s'il souhaite contrôler le positionnement des verres par rapport à la verticale.

## Revendications

1. Dispositif de saisie de l'écartement (e) existant entre la partie frontale du globe oculaire (4) de l'oeil d'un patient et la face interne (5a) d'un verre correcteur (5) fixé sur une monture de lunettes (3) portée par ledit patient, ledit dispositif comportant :
- une caméra (7) destinée à saisir une vue frontale de la monture (3) chaussée par le patient,
- des moyens d'analyse et de traitement d'images (9), destinés à calculer ledit écartement (e)
- un élément positionneur (1), destiné à être fixé à ladite monture (3), ledit élément positionneur (1) étant muni sur un côté de moyens de renvoi (15), destinés à renvoyer l'image transversale dudit écartement (e) en direction de l'objectif (7a) de la caméra (7) pour former, lorsque la monture (3) est fixée à l'élément positionneur (1) et est chaussée par le patient, sur une seule et même image, la vue frontale de la monture (3) équipée de l'élément positionneur (1), et la vue transversale montrant l'écartement (e).

2. Dispositif suivant la revendication 1, **caractérisé en ce que** les moyens de renvoi (15) sont fixés sur l'un des côtés latéraux de l'élément positionneur (1).

3. Dispositif suivant l'une des revendications 1 ou 2, **caractérisé en ce que** l'élément positionneur (1) est pourvu de deux repères décalés perpendiculairement à sa face frontale, à savoir un repère antérieur (16,20) et un repère postérieur (14, 21).

4. Dispositif suivant la revendication 3, **caractérisé en ce que** le repère postérieur (14,21) est disposé sur la face frontale de l'élément positionneur (1).

5. Dispositif suivant l'une des revendications 3 ou 4, **caractérisé en ce que** les repères antérieur (16,20) et postérieur (14,21) sont tels qu'ils permettent de caractériser au moins leur décalage dans le sens vertical.

6. Dispositif suivant la revendication 5, **caractérisé en ce que** les repères antérieur (16,20) et postérieur (14,21) sont tels qu'ils permettent de caractériser leur décalage dans le sens horizontal.

7. Dispositif suivant l'une des revendications 5 ou 6, **caractérisé en ce que** les repères (16, 20 ; 14,21) sont constitués de croix.

8. Dispositif suivant l'une des revendications précédentes, **caractérisé en ce que** les moyens de renvoi sont constitués d'un miroir plan (15) incliné d'un angle (α) voisin de 45° par rapport au plan de la monture de lunettes (3), de façon à renvoyer une image transversale dudit écartement (e) vers l'objectif (7a) de la caméra (7).

9. Dispositif suivant l'une des revendications 1 à 7, **caractérisé en ce que** les moyens de renvoi sont constitués d'un prisme à réflexion totale d'un angle au sommet voisin de 45° par rapport au plan de la monture de lunettes (3), de façon à renvoyer une image transversale dudit écartement vers l'objectif (7a) de la caméra (7).

10. Dispositif suivant l'une des revendications précédentes, **caractérisé en ce que** les moyens de renvoi (15) sont fixés de façon amovible sur l'élément positionneur (1).

## Claims

1. An input device of the clearance (e) located between the front part of the eyeball (4) of the eye of a patient and the inner face (5a) of a corrective lens (5) fixed on a spectacle frame (3) worn by said patient, said device comprising:
- a camera (7) designed to take a front view of the frame (3) worn by the patient,
- means for image analysis and processing (9) designed to calculate said clearance (e),
- a positioning element (1) designed to be fixed to said frame (3), said positioning element (1) being fitted on one side of the return means (15), designed to send the transversal image of said clearance (e) in the direction of the lens (7a) of the camera (7) to form on one and same image, when the frame (3) is fixed to the positioning element (1) and is worn by the patient, the front view of the frame (3) equipped with the positioning element (1), and the transversal view showing the clearance (e).

2. The device as claimed in Claim 1 **characterised in that** the return means (15) are fixed to one of the lateral sides of the positioning element (1).

3. The device as claimed in any one of Claims 1 or 2, **characterised in that** the positioning element (1) is provided with two marks offset perpendicularly to the front face, specifically an anterior mark (16, 20) and a posterior mark (14, 21).

4. The device as claimed in Claim 3, **characterised in that** the posterior mark (14, 21) is arranged on the front face of the positioning element (1).

5. The device as claimed in any one of Claims 3 or 4, **characterised in that** the anterior (16, 20) and posterior (14, 21) marks are such that they characterise their offset at least in the vertical direction.

6. The device as claimed in Claim 5, **characterised in that** the anterior (16, 20) and posterior (14, 21) marks are such that they characterise their offset at least in the horizontal direction.

7. The device as claimed in any one of Claims 5 or 6, **characterised in that** the marks (16, 20; 14, 21) are constituted by a cross.

8. The device as claimed in any one of the preceding claims, **characterised in that** the return means are constituted by a plane mirror (15) inclined at an angle (α) close to 45° relative to the plane of the spectacle frame (3) for sending a transversal image of said clearance (e) to the lens (7a) of the camera (7).

9. The device as claimed in any one of Claims 1 to 7, **characterised in that** the return means are constituted by a total reflection prism of an angle at the apex close to 45° relative to the plane of the spectacle frame (3) for sending a transversal image of said clearance (e) to the lens (7a) of the camera (7).

10. The device as claimed in any one of the preceding claims, **characterised in that** the return means (15) are fixed detachably on the positioning element (1).

## Patentansprüche

1. Vorrichtung zur Erfassung des Abstands (e), der zwischen dem vorderen Teil des Augapfels (4) des Auges eines Patienten und der Innenfläche (5a) einer Korrekturlinse (5) vorhanden ist, die auf einem vom Patienten getragenen Brillengestell (3) befestigt ist, wobei die Vorrichtung aufweist:
- eine Kamera (7), die dazu bestimmt ist, eine Vorderansicht des vom Patienten aufgesetzten Gestells (3) zu erfassen,
- eine Einrichtung zur Analyse und Verarbeitung von Bildern (9), die dazu bestimmt ist, den Abstand (e) zu berechnen,
- ein Positionierelement (1), das dazu bestimmt ist, am Gestell (3) befestigt zu werden, wobei das Positionierelement (1) auf einer Seite mit einer Reflexionseinrichtung (15) versehen ist, die dazu bestimmt ist, das Transversalbild des Abstands (e) in Richtung des Objektivs (7a) der Kamera (7) zu reflektieren, um, wenn das Gestell (3) am Positionierelement (1) befestigt ist und vom Patienten aufgesetzt ist, in ein- und demselben Bild die Vorderansicht des Gestells (3), das mit dem Positionierelement (1) ausgerüstet ist, und die den Abstand (e) zeigende Transversalansicht zu erzeugen.

2. Vorrichtung nach Anspruch 1, **dadurch gekennzeichnet, dass** die Reflexionseinrichtung (15) auf einer der Seitenflächen des Positionierelementes (1) befestigt ist.

3. Vorrichtung nach einem der Ansprüche 1 oder 2, **dadurch gekennzeichnet, dass** das Positionierelement (1) mit zwei Markierungen versehen ist, die in senkrechter Richtung versetzt zu seiner Vorderfläche angeordnet sind, und zwar einer vorderen Markierung (16, 20) und einer hinteren Markierung (14, 21).

4. Vorrichtung nach Anspruch 3, **dadurch gekennzeichnet, dass** die hintere Markierung (14, 21) auf der Vorderfläche des Positionierelementes (1) angeordnet ist.

5. Vorrichtung nach einem der Ansprüche 3 oder 4, **dadurch gekennzeichnet, dass** die vordere (16, 20) und die hintere Markierung (14, 21) derart beschaffen sind, dass sie zumindest eine Bestimmung einer Versetzung in vertikaler Richtung gestatten.

6. Vorrichtung nach Anspruch 5, **dadurch gekennzeichnet, dass** die vordere (16, 20) und die hintere Markierung (14, 21) derart beschaffen sind, dass sie eine Bestimmung ihrer Versetzung in horizontaler Richtung gestatten.

7. Vorrichtung nach einem der Ansprüche 5 oder 6, **dadurch gekennzeichnet, dass** die Markierungen (16, 20; 14, 21) aus Kreuzen besteht.

8. Vorrichtung nach einem der vorhergehenden Ansprüche, **dadurch gekennzeichnet, dass** die Reflexionseinrichtung aus einem Planspiegel (15) besteht, der um einen Winkel (α) von ca. 45° bezüglich der Ebene der Brillenfassung (3) geneigt ist, so dass ein Transversalbild des Abstands (e) zum Objektiv (7a) der Kamera (7) reflektiert wird.

9. Vorrichtung nach einem der Ansprüche 1 bis 7, **dadurch gekennzeichnet, dass** die Reflexionseinrichtung aus einem Totalreflexionsprisma mit einem Spitzenwinkel von ca. 45° bezüglich der Ebene der Brillenfassung (3) besteht, so dass ein Transversalbild des Abstands zum Objektiv (7a) der Kamera (7) reflektiert wird.

10. Vorrichtung nach einem der vorhergehenden Ansprüche, **dadurch gekennzeichnet, dass** die Reflexionseinrichtung (15) in unbeweglicher Weise am Positionierelement (1) befestigt ist.
